# EUROPEAN PATENT APPLICATION

(11) **EP 1 225 217 A1**
(43) Date of publication of application: **24.07.2002**
(21) Application number: 00966485.5
(22) Date of filing: 13.10.2000
(51) Int. Cl.: C12N 1/21, C12N 9/10, C12N 15/54

(54) **PROCESS FOR PRODUCING MICROORGANISM-ORIGIN TRANSGLUTAMINASE**

(30) Priority: 18.10.1999 JP 29564999
(71) Applicant: Ajinomoto Co., Inc., Tokyo 104-0031 (JP)
(72) Inventor: TAGUCHI, Seiichi, Kawagoe-Shi, Saitama 350-1123 (JP); MOMOSE, Haruo, Kamakura-Shi, Kanagawa 247-0071 (JP)
(74) Representative: HOFFMANN - EITLE
(86) International application number: JP0007135
(87) International publication number: WO0129187

(57) **Abstract**

The present invention relates to a method of secretory production of transglutaminase by a microorganism.

The object of the present invention is to provide a method of produce a large amount of transglutaminase by causing Streptomyces bacteria to produce and secrete a large amount of transglutaminase.

The present invention is a method of producing a large amount of transglutaminase, comprising culturing a Streptomyces bacterium harboring an expression plasmid containing a transglutaminase gene from actynomycetes and its native (naturally occurring) promoter, causing the bacterium to secrete protransglutaminase during the initial phase to the middle phase of culturing, and obtaining mature transglutaminase (active form) by cleaving and removing the pro9-structure, for example, with proteases derived from Streptomyces.

## Description

### Field of the invention

The present invention relates to a method for secretory production of actynomycetes-derived transglutaminase by genetic recombinant techniques using the host-vector system of Streptomyces lividans (also referred to as S. lividans hereinafter. Transglutaminase). Transglutaminase produced and secreted according to the present invention is widely used in the food processing or medicines.

Transglutaminase which is produced and secreted according to the present invention is an enzyme which catalyzes the acyl transfer reaction of γ-calboxylamide groups located in the peptide chain of proteins. When the enzyme is reacted with a protein, ε-(γ-Glu)-Lys cross linking reaction, the substitution reaction from Gln to Glu by deamination of Gln can be occurred. Transglutaminase is used for producing gelatinized foods such as jelly, or yogurt, cheese, gelatinized cosmetics or improving the quality of meat (Publication of unexamined Japanese patent application (referred to as JP-Kokai hereinafter) No. 1-50382). It is also used for producing materials for thermostable microcapsules or carriers for immobilized enzymes. Thus transglutaminase is an enzyme highly useful in the industry.

It is previously know that there are animal-derived transglutaminase and bacteria-derived transglutaminase (microbial transglutaminase, which may be referred to as MTG herein after). The former is a calcium dependent enzyme and distributes in the animal organs, skin or blood and the like. The examples are, for example, human keratinocyte transglutaminase (M. A. Phillips et al. Proc. Natl. Acad. Sci. USA, 87, 9333 (1990)), human blood coagulation factor XIII (A. Ichinose et al., Biochemistry, 25, 6900 (1990)). Regarding to the latter, calcium independent one is discovered from Streptoverticillium bacteria. The examples are, for example, Streptoverticillium griseocarneum IFO 12776, Streptoverticillium cinnamoneum sub sp. cinnamoneum (which may be referred to as S. cinnamoneum herein after) IFO 12852 and Streptoverticillium mobaraense (which may be referred to as S. mobaraense hereinafter) IFO 13819 (JP-Kokai No. 64-27471). Peptide mapping and genetic structural analysis revealed that transglutaminases produced by these bacteria have no homology with the enzymes from animals (EP 0 481 504 A1).

Microbial transglutaminases (MTGs) had problems in the amount or yield because they are produced through purification procedures from the culture of bacteria such as above described bacteria. The production of transglutaminase using genetic engineering techniques is also attempted. Transglutaminase proteins and genes thereof are described in , for example, JP-Kokai No. 64-27471, Biosci. Biotech. Biochem., 58, 82-87 (1994), Biosci. Biotech. Biochem., 58, 88-92 (1994), JP-Kokai No. 5-199883, Biochimie, 80, 313-319 (1998), Eur. J. Biochem., 257, 570-576 (1998), WO 9606931 and WO 9622366, which describe the reports for the production by host-vector system such as S. lividans, Aspergillus oryzae or Escherichia coli (which may be referred to as E. coli hereinafter). A producing method using secretory expression in microorganisms such as E. coli or yeast (JP-Kokai No. 5-199883) and a method for producing functional MTG by expressing MTG as an inactive fused protein inclusion body, resolubilizing the protein inclusion body by protein denaturing agent and then reconstituting the protein (JP-Kokai No.6-30771) are reported. However, in the secretory expression by conventional techniques using microorganisms, the problem of very poor expression level has been indicated. For secretory production of transglutaminase in Streptomyces, there is an report which describes an example including the specific description about secretory accumulation (Biosci. Biotech. Biochem., 58, 82-87 (1994); JP-Kokai No.5-199883), where transglutaminase gene from Streptoverticillium mobaraense was introduced into Streptoverticillium lividans as a host using genetic recombination techniques, however, the amount of the secretion was only about 0.1mg/l.

### Summary of the invention

It is an object of the present invention to provide a method of producing a large amount of transglutaminase by secretory production of transglutaminase in Streptomyces bacteria.

The method of the present invention is the method of producing a large amount or transglutaminase characterized in that a host-vector system for Streptomyces is used, where an expression plasmid which can highly express a gene in Streptomyces bacteria is constructed using transglutaminase gene from Streptomyces, that is, the signal peptide region and the pro-structural region and mature structural region and the native (natural) promoter region controlling the expression of transglutaminase, and the expression plasmid is introduced into a Streptomyces bacterium, the bacterium is cultured, and the bacterium is directed to secrete transglutaminase having additional pro-structural part (protransglutaminase) during the initial phase to the middle phase of culturing and then mature (active) transglutaminase is obtained in the late phase of culturing by cleaving and removing the pro-structure, for example, with proteases derived from Streptomyces.

### Brief description of Drawings

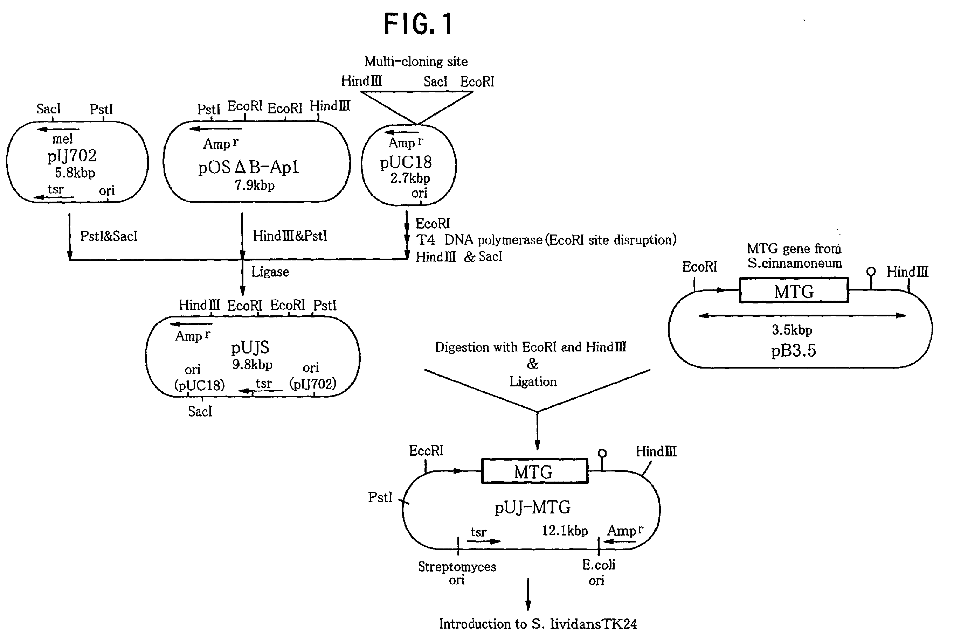

Figure 1. shows the construction procedure of the expression plasmid pUJ-MTG.

### Detailed Description of the Invention

According to the present invention, a large amount of mature (active form) transglutaminase are obtained by using a Streptomyces bacterium as a host-vector system and generating an expression construct containing the gene for transglutaminase having the pro-structural part (pro-transglutaminase) linked to the down stream of the native promoter for transglutaminase gene, and by introducing the construct into a Streptomyces bacterium, expressing it and cleaving the extracellulary secreted pro-transglutaminase, for example, with proteases produced by the same Streptomyces bacterium.

Secretory proteins are generally known to be translated as pre-peptides or prepro-peptides and then undergo post-translational modifications to generate mature proteins. Namely, it is generally known that secretory proteins are translated as pre-peptides or prepro-peptides, and then, the signal peptides (pre-part) thereof are cleaved to convert them into mature peptides or pro-peptides and the pro-parts of the pro-peptides are cleaved to generate the mature peptides. Transglutaminase is one of such proteins. As used herein, transglutaminase having both the signal peptide and the pro-part, namely the primary translational product, may be referred to as "prepro-transglutaminase" and transglutaminase having the pro-part but not the signal peptide may be referred to as "pro-transglutaminase". The pro-part of pro-transglutaminase may be referred to as "pro-structural part" or simply "pro-structure". As used herein, the "pro-structural part/pro-structure" of transglutaminase and the "pro-part" of the protein are used interchangeably. Thus, "pro-transglutaminase" may also be referred to as "transglutaminase with additional pro-structural part".

As used herein, a protein of which pro-part is "cleaved and removed" refer to a protein wherein one or more amino acid constituting the pro-part is removed by cleaving peptide bond, and it includes a protein having the identical N-terminal region to the native mature protein and also a protein having one or more additional amino acid derived from the pro-part at its N-terminal compared to the native protein or a protein having shorter amino add sequence than that of the native mature protein. As used herein, "mature transglutaminase" and "active form transglutaminase" are use in the same meaning.

Generally, the genetic constructs used in the present invention are those having suitable sequences containing promoters, nucleotide fragments encoding prepro-transglutaminase and regulatory sequences required to express the intended proteins in Streptomyces bacteria, at the appropriate location. The Vector which can be used for the constructs are not limited and any vector can be used which can function in Streptomyces bacteria, and the vectors may be vectors which autonomously replicate extrachromosomally such as plasmids or may be vectors which can integrated into the bacterial chromosome. Plasmids from Streptomyces are preferable, and the examples include, for example, plJ702 (J. Gen. Microbiol., 129, 2703-2714, (1983)) and the plasmids obtained by improving it.

The promoters which can be used in the present invention to express transglutaminase genes in Streptomyces bacteria are the native promoters of transglutaminase genes from Actinomycetes. However, in some cases the promoter region cannot be identified, because the consensus sequence is not established in Actinomycetes in contrast with E.coli. In such cases, the genetic fragment containing 5'-upstream region large enough to covering the structural gene for transglutaminase and the promoter region required to regulate its expression may be used.

Transglutaminase genes which can be used in the present invention are not particularly limited, and, for example, genes for secretory transglutaminase derived from S. cinnamoneum IFO 12852, S. grosepcarneum IFO 12776, S. mobaraence IFO 13819 and Streptoverticillium lydicus (WO96/06931) are preferable. Transglutaminase genes from S. cinnamoneum or S. mobaraense are particularly preferable, which are used with the native promoter of the respective transglutaminase genes.

The entire nucleotide sequence of transglutaminase gene from S. cinnamoneum IFO12852 containing the 5'-upstream region, which was identified by the inventors of the present invention, is shown in SEQ ID NO:1, and the amino acid sequence encoded by the nucleotide sequence is shown in SEQ ID NO:2. It was assumed that from amino acid at position 1 to position 32 in the amino acid sequence was the sequence for pre-part, from amino acid at position 33 to position 86 was the sequence for pro-part and from amino acid at position 87 to position 416 was the sequence for mature transglutaminase. The entire nucleotide sequence of transglutaminase gene containing the 5'-upstream region from S. mobaraense is also shown in SEQ ID NO:3, and the amino acid sequence encoded by the nucleotide sequence is shown in SEQ ID NO:4. From amino acid at position 1 to position 31 in the amino acid sequence is the sequence for pre-part, from amino acid at position 32 to position 76 is the sequence for pro-part and from amino acid at position 77 to position 407 is the sequence for mature transglutaminase.

Methods for introducing the genetic constructs used in the present invention are not particularly limited, and the protoplast process (Gene, 39, 281-286 (1985); JP-Kokai No. 3-251182), electroporation process (Bio/Technology, 7, 1067-1070 (1989)) may be used. The thus obtained transformants may be cultured by using conventional methods and conditions. The medium for culturing these microorganisms may be a conventional medium, for example, a medium containing carbon sources, nitrogen sources and inorganic ions. It is preferable to add vitamins, organic micronutrients such as amino acids or natural materials such as polypeptone or yeast extract. As carbon sources, carbohydrates such as solubilized starch, glucose or sucrose, organic acids and alcohols are properly used. The culture is conducted under the aerobic condition for one day to 2 weeks appropriately maintaining pH ranging from 5.0 to 8.5 and the temperature ranging from 15°C to 37°C.

As nitrogen sources, ammonia gas, aqueous ammonia, ammonium salts and the like are used. As inorganic ions, magnesium ion, phosphate ion, potassium ion or iron ions and the like are properly used. By culturing the transformants under these conditions, prepro-transglutaminase is largely produced in the bacterial cells and extracellularly secreted as protransglutaminase and then pro-transglutaminase is cleaved in the medium by a protease which is produced and secreted by a Streptomyces bacterium itself under these condition, then mature (active form) transglutaminase is largely accumulated in the medium.

Transglutaminases produced and secreted according to the present invention can be purified from the medium after culturing, depending on their properties, using the methods well known to those skilled in the art. Transglutaminase may be purified, for example, by using known appropriate techniques such as ammonium sulfate precipitation or ethanol precipitation, as well as ion-exchange column chromatography, isoelectric focusing or gel filtration or the combination thereof, after removing cells, for example, by centrifugation.

### Examples

### Example 1: Acquisition of transglutaminase gene from S. cinnamoneum IFO 12852

The sequence of transglutaminase from S. cinnamoneum CBS683.68 has been already determined (Biochimie., 80, 313-319 (1998)). The primer according to SEQ ID NO:5 and SEQ ID NO:6 are synthesized based on this sequence, and the region encoding mature transglutaminase was amplified by PCR from chromosomal DNA of S. cinnamoneum IFO 12852 prepared according to the method of Saito and Miura (Biochem., biophys., Act., 72, 619 (1963)). PCR was conducted using Pyrobest DNA polymerase (TAKARA Co.) under the condition according to its protocol.

SEQ ID NO:5 5'-TCCGATGACCGGGAAACTCCTCCCGCCGAG-3'

SEQ ID NO:6 5'-CGGCCAGCCTTGCTCCACCTTGGCGGGGGC-3'

### [ Free text in the sequence listing]

SEQ ID NO:5 :PCR primer for amplifying transglutaminase gene from S. cinnamoneum
SEQ ID NO:6 :PCR primer for amplifying transglutaminase gene from S. cinnamoneum

The amplified 960bp DNA fragment are then reacted with [α-³²P]dCTP using Random Primer DNA Labeling Kit Ver.2 (Takara Co.), according to the protocol attached to the kit, to generate the DNA probe. Using the generated probe and the chromosomal DNA from S. cinnamoneum IFO 12852, Southern blot hybridization was conducted according to the conventional procedures such as those described in Molecular Cloning 2nd Edition (J. Sambrook, E. F. Fritsch and T. Maniatis, Cold Spring Harbor Laboratory Press, p9.31 (1989)), which confirmed that transglutaminase gene was present in the 3.5kb fragment which excised by BamHI restriction enzyme. The 3.5kb BamHI digested fragment from S. cinnamoneum IFO 12852 chromosomal DNA was recovered by agarose electrophoresis using EASYTRP Ver. 2 (Takara), inserted into BamHI site of pUC18, and introduced into competent Escherichia coli JM109 cells (Takara) to generate a library. Using the previously prepared DNA probe, the library was screened by colony hybridization according to the conventional procedures such as those described in Molecular Cloning 2nd Edition (J. Sambrook, E. F. Fritsch and T. Maniatis, Cold Spring Harbor Laboratory Press, p9.31 (1989)) to obtain the strain harboring the plasmid where the fragment of transglutaminase gene was cloned. The plasmid was recovered from the strain, which was designated as pB3.5.

The sequencing of the cloned fragment in pB3.5 confirmed that the transglutaminase gene from S. cinnamoneum IFO 12852 had almost identical nucleotide sequence to the transglutaminase gene from S. cinnamoneum CBS683.68. The transglutaminase gene was inserted such that the gene was transcribed in the direction from EcoRI to HindIII site within the multi-cloning site of pUC18. The sequencing was performed using Di-terminator Cycle Sequencing Kit (PE Applied Biosystems) and DNA Sequencer 373A (PE Applied Biosystems).

The determined nucleotide sequence and the amino acid sequence encoded by the nucleotide sequence are shown in SEQ ID NO:1 and SEQ ID NO:2, respectively. It was assumed that from amino acid at position 1 to position 32 in the amino acid sequence is the sequence for pre-part, from amino acid position 33 to position 86 is the sequence for pro-part and from amino acid at position 87 to position 416 is the sequence for mature transglutaminase.

### Example 2: Construction of the expression plasmid for transglutaminase gene

### 1) Acquisition of plasmid vector (plJ702)

plJ702 was prepared according to [J. Bacteriol., 162, 406-412 (1985); J. Bacteriol., 169, 1929-1937 (1987)]. More specifically, Streptomyces lividans 3131 (ATCC 35287)(J. Gen. Microbiol., 129, 2703-2714 (1983)) obtained by transforming Streptomyces lividans 66 with plJ702 was cultured under the following medium condition at 30°C for 2 days.

| [YEME medium + 0.5% Glycine + 50µg/ml thiostrepton] | |
|---|---|
| 0.3% | Yeast Extract |
| 0.5% | Peptone |
| 0.3% | Malto Extract |
| 0.1% | Magnesium chloride |
| 1.0% | Glucose |
| 34.0% | Sucrose |
| 0.5% | Glycine |
| 0.1% | 50mg/ml thiostrepton solution |
| | (Sigma : dimethylsulfoxide solution)(pH 7.0) |

200ml of the broth cultured under above condition was centrifuged (12,000g, 4°C, 10 min.), washed with 50mM Tris-HCI (pH(8.0) - 5mM EDTA-50mM NaCI, and the resulting bacterial cells were suspended in 10ml of Tris-HCI (pH8.0) - 10mM EDTA - 25% Sucrose ( TE-Sucrose). 2ml of TE-Sucrose containing 30mg/ml of Lysozyme (Sigma) and 4ml of 0.25M EDTA was added, the mixture was incubated at 37°C for 30 minutes, then 2ml of 20% SDS was added. 5ml of 5M NaCI was further added, gently mixed, and then, it was incubated overnight. After centrifugation (100,000g, 4°C, 40 min. ), 30% polyethylene glycol 6000 was added to the resulting supernatant at the final concentration of 10% and the mixture was incubated at 0°C for 4.5 hours. Then the mixture was centrifuged (900g, 4°C, 5 min.) and the resulting precipitation was dissolved in 10mM Tris-HCl (pH 8.0)-1mM EDTA - 50mM NaCI. To the mixture 1.2ml of the solution prepared to contain 16.8g of cesium chloride and 10mg/ml of ethidium bromide in 10mM Tris-HCI(pH8.0) - 1mM EDTA (referred to as "TE", hereinafter) was added, the residue was remove by centrifugation (1,300g, room temperature, 15 min.), and then the mixture was centrifuged (230,000g, 20°C, 12 hours). After centrifugation, the plasmid layer was drawn and isolated under UV lamp, the solution was repeatedly extracted with TE-saturated buthanol 3 times to remove ethidium bromide. The solution was dialyzed against TE at 4°C overnight, extracted once with TE-saturated phenol and twice with chloroform/isoamyl alcohol, and then the aqueous layer was recovered. Then, 1/10 volume of 3M sodium acetate (pH5.2) solution and 2 volume of ethanol were added to the solution, and the mixture was allowed to stand at -80°C for 30 minutes. The precipitation was recovered by centrifugation (12,0000g, 4°C, 15 minutes), washed with 70% ethanol, dried and dissolved in 200µl of TE. About 10µg of the plasmid was obtained.

### 2) Construction of the expression plasmid

A shuttle vector was firstly constructed which can replicate both in actinomycetes (Streptomyces) host and E.coli host. Multicopy plasmid plJ702 for actinomycetes was digested with Sacl and Pstl to prepare large 5.1kb mel (tyrosine kinase gene) fragment from which the promoter region was deleted. pOSΔB-Ap1 (about 7.9kb)(Appl. Environ, Microbiol., 60, 3566-3572 (1994)) into which the fusion gene of protease inhibitor SSI (Streptomyces subtilisin inhibitor) gene and microbial peptide (apidaecin) gene was digested with HindIII and Pstl to prepare the fragment of about 2kb. Multicopy plasmid pUC18 (Takara) for Escherichia coli was digested with EcoRI, blunted with T4 DNA polymerase (Takara), and self ligated. Plamids which could not be digested with EcoRI were selected and the plasmids were digested with SacI and HindIII to prepare the 2.7kb fragment. Then, shuttle vector pUJS (about 9.8kb) was constructed by the ternary ligation of the 5.1kb Sacl-Pstl fragment from pIJ702, the 2kb HindIII-PstI fragment from pOSΔB-Ap1 and the 2.7kb SacI-HindIII fragment from pUC18.

pUJS was digested with HindIII and EcoRI to recover the large 8.6kb fragment. pB3.5 (about 6.2kb) containing transglutaminase gene from S. cinnamoneum IFO 12852 which was cloned according to (1) was digested with HindIII and EcoRI and the 3.5kb HindIII-EcoRI fragment was recovered. The 3.5kb HindIII-EcoRI fragment was inserted into HindIII and EcoRI site of pUJS to construct pUJ-MTG (about 12.1kb). The above described construction procedures are shown in Figure 1.

E. coli AJ13669 obtained by transforming E.coli with pUJ-MTG was deposited in the National Institute of Bioscience and Human-Technology Agency of Industrial Science and Technology (1-3, Higashi 1 chome Tsukuba-shi Ibarakiken 305-8566, JAPAN) (The microorganism had been deposited as FERM P-17602 on October 14, 1999 and was transferred to the international deposit based on the Budapest Treaty as FERM BP-7287 on August 28, 2000).

### Example 3: Transformation of S. lividans TK24

S. lividans TK24 is the strain derived from S. lividans 66, which is mounted a streptomycin resistance (GENETIC MANIPULATION OF STREPTOMYCES, A LABORATORY MANYAL: D.A. Hopwood et al., p266, 1985, The john Innes Foundation Norwich). This strain was provided from D.A. Hopwood (John Innes Institute, Colney Lane, Norwich NR4 7UH, U.K.) and is obtainable from D.A. Hopwood's laboratory. S. lividans Tk24 was treated to make protoplasts and transformed according to the method of [JP-Kokai No. 3-251182; Hunter, I.S., "DNA Cloning" A Practical Approach 2, Glover, D.M.(Ed.) IRL Press (1985), GENETIC MANIPULATION OF STREPTOMYCES, A LABORATORY MANUAL: D.A. Hopwood et al., p104, 1985, The John Innes Foundation Norwich]. More specifically, S. lividans was cultured in YEME medium + 0.5% Glycine at 30°C for two(2) days. 200ml of broth was centrifuged(1,300g, room temperature, 10 min.) and the resulting bacterial cells were suspended in 72ml of 0.35M sucrose.

The suspension was then centrifuged (1,300g, room temperature, 10 min.), incubated at 30°C for 2.5 hours, filtrated with absorbent cotton to remove residues. The resulting filtrate was centrifuged (1,300g, room temperature, 10 min.) and the precipitant was washed twice with 25ml of P-buffer and suspended in 1ml of P-buffer to prepare a protoplast suspension.

| [P-buffer] | |
|---|---|
| TES[N-Tris(hydroxymethyl)methyl-2-aminoethane sulphonic acid] | 5.73g |
| Sucrose | 103g |
| Magnesium chloride | 2.03g |
| Potassium sulfate | 0.5g |
| Calcium chloride | 3.68g |
| Trace elements solution | 2ml/L (pH7.4) |

1ml of 1% potassium phosphate solution per 100ml of P-buffer, which had been separately prepared, was added to P-buffer immediately before use.

### [Trace elements solution]

It contains the followings per 1L of the solution:

| | |
|---|---|
| Zinc chloride | 40mg |
| Ferric chloride | 200mg |
| Cupric chloride | 10mg |
| Manganese chloride | 10mg |
| Tetra sodium borate | 10mg |
| ammonium molybdate | 10mg |

The transformation of S. lividans TK24 protoplast suspension with pUJ-MTG (about 12.1kb), transglutaminase gene expressing plasmid, was conducted as follows.

| | |
|---|---|
| DNA solution (0.2µg/µl) | 20µl |
| Protoplast suspension of S. lividans TK24 | 100µl |
| 0.35M Sucrose | 20µl |
| P-buffer containing 20% polyethylene glycol 1000 | 1.5ml |

was gently mixed and allowed to stand for 2 minutes at the room temperature.

The mixture was centrifuged (1,700g, room temperature, 10 min.) and the pellet was collected, repeatedly washed twice with P-buffer, suspended in 1ml of P-buffer and spreaded on the following R-2 agar plates.

### [R-2 Agar plate]

1) R-2/A

| | |
|---|---|
| Potassium sulfate | 0.5g/l |
| Magnesium chloride | 20.2g/l |
| Calcium chloride | 5.9g/l |
| Glucose | 20.0g/l |
| Proline | 6.0g/l |
| Casamino acid | 0.2g/l |
| Trace elements solution | 4ml |
| Agar | 44.0g/l |

2) R-2/B

| | |
|---|---|
| TES | 11.5g/l |
| Yeast Extract | 10.0g/l |
| Sucrose | 203g/l (pH7.4) |

3) 1% KH₂PO₄
1), 2) and 3) were separately prepared. R-2/A and R-2/B were mixed on the preparation of plates and 1ml of 1% KH₂PO₄ solution per final volume of 200ml of the mixture was added. R-2 agar plates where the transformants were plated were incubated at 30°C for 18 hours. 1ml of P-buffer containing 200µg/ml of thiostrepton was poured on the pate to cover the entire surface of the agar and the plates were further incubated at 30°C for 7 days to obtain colonies. Plasmids were prepared from the obtained colonies to confirm that the intended plasmid was introduced.

### Example 4: The expression of transglutaminase and secretory production

The transformant pUJ-MTG/S.lividans TK24 was cultured in 4ml of Tripton-Soya Broth (DIFCO) liquid medium containing 10µg/ml of thiostrepton at 30°C for 3 days. 1ml of the culture was seeded into 100ml of the same liquid medium in 500ml Sakaguchi flask and cultured at 30°C for 2 weeks. The samples were taken sequentially from the culture and 10µl of the broth supernatant was subjected to SDS-PAGE and then to Western blot analysis using the anti-transglutaminase antibody described in JP-Kokai No.6-046855 according to the general method such as described in Molecular Cloning 2nd Edition (J. Sambrook, E.F. Fritsch and T. Maniatis, Cold Spring Harbor Laboratory Press, p18.60 (1989)). As a result, the secretory production (about 40-50mg/l) of transglutaminase with additional pro-structural part was found till about day 7 to 10 of culturing and then the yield of transglutaminase having almost the same molecular weight as that of mature transglutaminase, which can be the result of processing of pro-transglutaminase, increased on further culturing. At about week 2 in culturing, about 40-50mg/l of mature transglutaminase was accumulated.

Using the supernatant of broth at the phase where the amount of secretory production of transglutaminase with additional pro-structural part (protransglutaminase) was large, SDS-PAGE and semi-dry blotting to PVDF membrane were conducted ( Analysis of Protein Structure for Gene Cloning, Tokyo Kagaku Dojin (1993)). After blotting, the PVDF membrane was stained with Coomassie brilliant blue, de-stained and dried in air. The portion corresponding to pro-transglutaminase was excised and analyzed for the N-terminal amino acid sequence by the protein sequencer (Model 476A, Perkin-Elmer). The result confirmed the 10 amino acids sequence (Gly-Asp-Gly-Glu-Glu-Lys-Gly-Ser-Tyr-Ala-, SEQ ID NO:7) of pro-transglutaminase. This amino acid sequence differed from the sequence of pro-region indicated in Biochimie., 80, 313-319 (1998), but was identical to the amino acid sequence determined in Example 1 (SEQ ID NO:2).

According to the present invention, a large amount of transglutaminase can be obtained in the broth by directing Streptomyces bacteria to produce transglutaminase. Since transglutaminase accumulated in the broth according to the present invention is mature transglutaminase cleaved by proteases which are produced by the Streptomyces bacteria themselves, mature transglutaminase can be easily recovered from the broth on a large scale.

## Claims

1. A bacterium belonging to Streptomyces, wherein a gene construct containing a transglutaminase gene from actinomycetes and a promoter sequence controlling the transglutaminase gene is introduced into the bacterium.

2. The bacterium belonging to Streptomyces according to claim 1, wherein the actinomycetes is Streptoverticillium cinnamoneum.

3. The bacterium belonging to Streptomyces according to claim 1 or 2, wherein the bacterium belonging to Streptomyces is Streptomyces lividans.

4. A method of producing a pro-transglutaminase derived from actinomycetes, comprising the steps of culturing the bacterium belonging to Streptomyces according to any one of claims 1 to 3, and allowing the bacterium to secrete the pro-transglutaminase into a culture medium.

5. A method of producing a mature transglutaminase derived from actinomycetes, comprising the steps of culturing the bacterium belonging to Streptomyces according to any one of claims 1 to 3, allowing the bacterium to secrete a protransglutaminase derived from actinomycetes into a culture medium, cleaving the pro-structural part of the pro-transglutaminase by a protease derived from said bacterium belonging to Streptomyces and recovering the mature transglutaminase.

6. The method according to claim 4, wherein the pro-transglutaminase has the amino acid sequence from Glycine residue at position 33 to Proline residue at position 416 in the sequence of SEQ ID NO:2.

7. The method according to claim 5, wherein the mature transglutaminase has the amino acid sequence from Serine residue at position 87 to Proline residue at position 416 in the sequence of SEQ ID NO:2.


SEQUENCE LISTING

<110> Ajinomoto Co., Inc.

<120> A method of producing microbial transglutaminase

<130> Y1H0984

<140>
<141>

<150> JP 11-295649
<151> 1999-10-18

<160> 7

<170> PatentIn Ver. 2.1

<210> 1
<211> 1461
<212> DNA
<213> Streptoverticillium cinnamoneum

<220>
<221> CDS
<222> (151)..(1398)

<400> 1
cggcggcagc cctccttgcc gccggcgcag cgacgcagga cggcgcggcc aaggccctga 60

gcggcagctc gtcgcaaacc cctccatcgc gtcgtgctct cacatgccct cgtttcacga 120

ggcttcacca caagggagtt attgatttcc atg cac aaa cgt cgg aga ctt ctc 174
Met His Lys Arg Arg Arg Leu Leu
1               5

gcc ttc gcc act gtg ggt gcg gtc ata tgc acc gca gga ttc aca cct 222
Ala Phe Ala Thr Val Gly Ala Val Ile Cys Thr Ala Gly Phe Thr Pro
    10                  15                  20

tcg gtc agc cag gcc gcc agc agt ggc gat ggg gaa gag aag ggg tcc 270
Ser Val Ser Gln Ala Ala Ser Ser Gly Asp Gly Glu Glu Lys Gly Ser
25                  30                  35                  40

tac gcc gaa acg cac ggc ctg acg gcg gat gac gtc gag agc atc aac 318
Tyr Ala Glu Thr His Gly Leu Thr Ala Asp Asp Val Glu Ser Ile Asn
                45                  50                  55

gca ctg aac gaa aga gct ctg act ctg ggc caa cct ggc aag cct ccg 366
Ala Leu Asn Glu Arg Ala Leu Thr Leu Gly Gln Pro Gly Lys Pro Pro
            60                  65                  70

aag gaa tta cct ccg agc gcc agc gcg ccc tcc cgg gcc ccc tcc gat 414
Lys Glu Leu Pro Pro Ser Ala Ser Ala Pro Ser Arg Ala Pro Ser Asp
        75                  80                  85

gac cgg gaa act cct ccc gcc gag ccg ctc gac agg atg cct gag gcg 462
Asp Arg Glu Thr Pro Pro Ala Glu Pro Leu Asp Arg Met Pro Glu Ala

    90                  95                  100

tac cgg gcc tac gga ggc agg gcc act acg gtc gtc aac aac tac ata 510
Tyr Arg Ala Tyr Gly Gly Arg Ala Thr Thr Val Val Asn Asn Tyr Ile
105                 110                 115                 120

cgc aag tgg cag cag gtc tac agt cac cgc gac gga aag aaa cag caa 558
Arg Lys Trp Gln Gln Val Tyr Ser His Arg Asp Gly Lys Lys Gln Gln
                125                 130                 135

atg acc gaa gag cag cga gaa aag ctg tcc tac ggt tgc gtt ggc gtc 606
Met Thr Glu Glu Gln Arg Glu Lys Leu Ser Tyr Gly Cys Val Gly Val
            140                 145                 150

acc tgg gtc aac tcg ggc ccc tac ccg acg aac aga ttg gcg ttc gcg 654
Thr Trp Val Asn Ser Gly Pro Tyr Pro Thr Asn Arg Leu Ala Phe Ala
        155                 160                 165

tcc ttc gac gag aac aag tac aag aac gac ctg aag aac acc agc ccc 702
Ser Phe Asp Glu Asn Lys Tyr Lys Asn Asp Leu Lys Asn Thr Ser Pro
    170                 175                 180

cga ccc gat gaa acg cgg gcg gag ttc gag ggt cgc atc gcc aag ggc 750
Arg Pro Asp Glu Thr Arg Ala Glu Phe Glu Gly Arg Ile Ala Lys Gly
185                 190                 195                 200

agt ttc gac gag ggg aag ggt ttc aag cgg gcg cgt gat gtg gcg tcc 798
Ser Phe Asp Glu Gly Lys Gly Phe Lys Arg Ala Arg Asp Val Ala Ser
                205                 210                 215

gtc atg aac aag gcc ctg gaa aat gcc cac gac gag ggg act tac atc 846
Val Met Asn Lys Ala Leu Glu Asn Ala His Asp Glu Gly Thr Tyr Ile
            220                 225                 230

aac aac ctc aag acg gag ctc acg aac aac aat gac gct ctg ctc cgc 894
Asn Asn Leu Lys Thr Glu Leu Thr Asn Asn Asn Asp Ala Leu Leu Arg
        235                 240                 245

gag gac agc cgc tcg aac ttc tac tcg gcg ctg agg aac aca ccg tcc 942
Glu Asp Ser Arg Ser Asn Phe Tyr Ser Ala Leu Arg Asn Thr Pro Ser
    250                 255                 260

ttc aag gaa agg gac ggc ggc aac tac gac ccg tcc aag atg aag gcg 990
Phe Lys Glu Arg Asp Gly Gly Asn Tyr Asp Pro Ser Lys Met Lys Ala
265                 270                 275                 280

gtg atc tac tcg aag cac ttc tgg agc ggg cag gac cag cgg ggc tcc 1038
Val Ile Tyr Ser Lys His Phe Trp Ser Gly Gln Asp Gln Arg Gly Ser
                285                 290                 295

tcc gac aag agg aag tac ggc gac ccg gaa gcc ttc cgc ccc gac cag 1086
Ser Asp Lys Arg Lys Tyr Gly Asp Pro Glu Ala Phe Arg Pro Asp Gln
            300                 305                 310

ggt acc ggc ctg gtc gac atg tcg aag gac aga agc att ccg cgc agt 1134
Gly Thr Gly Leu Val Asp Met Ser Lys Asp Arg Ser Ile Pro Arg Ser
        315                 320                 325

ccg gcc aag ccc ggc gaa ggt tgg gtc aat ttc gac tac ggt tgg ttc 1182
Pro Ala Lys Pro Gly Glu Gly Trp Val Asn Phe Asp Tyr Gly Trp Phe
    330                 335                 340

ggg gct caa aca gaa gcg gat gcc gac aaa acc aca tgg acc cac ggc 1230
Gly Ala Gln Thr Glu Ala Asp Ala Asp Lys Thr Thr Trp Thr His Gly
345                 350                 355                 360

gac cac tac cac gcg ccc aat agc gac ctg ggc ccc atg cac gta cac 1278
Asp His Tyr His Ala Pro Asn Ser Asp Leu Gly Pro Met His Val His
                365                 370                 375

gag agc aag ttc cgg aag tgg tct gcc ggg tac gcg gac ttc gac cgc 1326
Glu Ser Lys Phe Arg Lys Trp Ser Ala Gly Tyr Ala Asp Phe Asp Arg
            380                 385                 390

gga gcc tac gtg atc acg ttc ata ccc aag agc tgg aac acc gcc ccc 1374
Gly Ala Tyr Val Ile Thr Phe Ile Pro Lys Ser Trp Asn Thr Ala Pro
        395                 400                 405

gcc aag gtg gag caa ggc tgg ccg tgacaggctg gtactacgac ctctgctgat 1428
Ala Lys Val Glu Gln Gly Trp Pro
    410                 415

ttctgcccgg tcagtccacg cctctcgacg cga 1461

<210> 2
<211> 416

<212> PRT
<213> Streptoverticillium cinnamoneum

<400> 2
Met His Lys Arg Arg Arg Leu Leu Ala Phe Ala Thr Val Gly Ala Val
1               5                   10                  15

Ile Cys Thr Ala Gly Phe Thr Pro Ser Val Ser Gln Ala Ala Ser Ser
            20                  25                  30

Gly Asp Gly Glu Glu Lys Gly Ser Tyr Ala Glu Thr His Gly Leu Thr
        35                  40                  45

Ala Asp Asp Val Glu Ser Ile Asn Ala Leu Asn Glu Arg Ala Leu Thr
    50                  55                  60

Leu Gly Gln Pro Gly Lys Pro Pro Lys Glu Leu Pro Pro Ser Ala Ser
65                  70                  75                  80

Ala Pro Ser Arg Ala Pro Ser Asp Asp Arg Glu Thr Pro Pro Ala Glu
                85                  90                  95

Pro Leu Asp Arg Met Pro Glu Ala Tyr Arg Ala Tyr Gly Gly Arg Ala
            100                 105                 110

Thr Thr Val Val Asn Asn Tyr Ile Arg Lys Trp Gln Gln Val Tyr Ser
        115                 120                 125

His Arg Asp Gly Lys Lys Gln Gln Met Thr Glu Glu Gln Arg Glu Lys

    130                 135                 140

Leu Ser Tyr Gly Cys Val Gly Val Thr Trp Val Asn Ser Gly Pro Tyr
145                 150                 155                 160

Pro Thr Asn Arg Leu Ala Phe Ala Ser Phe Asp Glu Asn Lys Tyr Lys
                165                 170                 175

Asn Asp Leu Lys Asn Thr Ser Pro Arg Pro Asp Glu Thr Arg Ala Glu
            180                 185                 190

Phe Glu Gly Arg Ile Ala Lys Gly Ser Phe Asp Glu Gly Lys Gly Phe
        195                 200                 205

Lys Arg Ala Arg Asp Val Ala Ser Val Met Asn Lys Ala Leu Glu Asn
    210                 215                 220

Ala His Asp Glu Gly Thr Tyr Ile Asn Asn Leu Lys Thr Glu Leu Thr
225                 230                 235                 240

Asn Asn Asn Asp Ala Leu Leu Arg Glu Asp Ser Arg Ser Asn Phe Tyr
                245                 250                 255

Ser Ala Leu Arg Asn Thr Pro Ser Phe Lys Glu Arg Asp Gly Gly Asn
            260                 265                 270

Tyr Asp Pro Ser Lys Met Lys Ala Val Ile Tyr Ser Lys His Phe Trp
        275                 280                 285

Ser Gly Gln Asp Gln Arg Gly Ser Ser Asp Lys Arg Lys Tyr Gly Asp
    290                 295                 300

Pro Glu Ala Phe Arg Pro Asp Gln Gly Thr Gly Leu Val Asp Met Ser
305                 310                 315                 320

Lys Asp Arg Ser Ile Pro Arg Ser Pro Ala Lys Pro Gly Glu Gly Trp
325 330 . 335

Val Asn Phe Asp Tyr Gly Trp Phe Gly Ala Gln Thr Glu Ala Asp Ala
340 345 350

Asp Lys Thr Thr Trp Thr His Gly Asp His Tyr His Ala Pro Asn Ser
355 360 365

Asp Leu Gly Pro Met His Val His Glu Ser Lys Phe Arg Lys Trp Ser
370 375 380

Ala Gly Tyr Ala Asp Phe Asp Arg Gly Ala Tyr Val Ile Thr Phe Ile
385 390 395 400

Pro Lys Ser Trp Asn Thr Ala Pro Ala Lys Val Glu Gln Gly Trp Pro
405 410 415

<210> 3
<211> 1809
<212> DNA

<213> Streptoverticillium mobaraense

<220>
<221> CDS
<222> (578)..(1798)

<400> 3
gtcgacgcgg gccgggaggg ggtgcggcgg cgcccttcgg ctgtgtggac gaagcgtcgg 60
gtcggagggg cggccggata tcgtccttgg ggcggggtgg ccggaattgc cgccatggtg 120
ttgccgggga atcgacccga agacatgatc acttctcgta tccacccgat cacgtatccg 180
ggagtcgaga agtgttacgc cgtgcccctg tccgcgtcct cacccctgtc gccgtgacag 240
cgacccgcgt tcttccactc gcacggacgg ccccacagga cctttcggcc cgggctcgcc 300
ccgccgcctc ggtgacggcc tccgaataac gcggccgccg gggcctcggc cggttgaccg 360
atccgggtca cgcgccccgc cgggcgggcg gccacgtccg gtctcgcccc gcccgacatc 420
ggctgcgact gccttcgctc gcacttcttc ccgcctcccg gccgcgtttt tccgccgccg 480
aaggtgcggc gacgcgtacc gaatccccct tcatcgcgac gtgcttccgc acggccgcgt 540
tcaacgatgt tccacgacaa aggagttgca ggtttcc atg cgc ata cgc cgg aga 595
gct ctc gtc ttc gcc act atg agt gcg gtg tta tgc acc gcc gga ttc 643
atg ccg tcg gcc ggc gag gcc gcc gcc gac aat ggc gcg ggg gaa gag 691
acg aag tcc tac gcc gaa acc tac cgc ctc acg gcg gat gac gtc gcg 739
aac atc aac gcg ctc aac gaa agc gct ccg gcc gct tcg agc gcc ggc 787
ccg tcg ttc cgg gcc ccc gac tcc gac gac agg gtc acc cct ccc gcc 835
gag ccg ctc gac agg atg ccc gac ccg tac cgt ccc tcg tac ggc agg 883
gcc gag acg gtc gtc aac aac tac ata cgc aag tgg cag cag gtc tac 931
agc cac cgc gac ggc agg aag cag cag atg acc gag gag cag cgg gag 979
tgg ctg tcc tac ggc tgc gtc ggt gtc acc tgg gtc aat tcg ggt cag 1027
tac ccg acg aac aga ctg gcc ttc gcg tcc ttc gac gag gac agg ttc 1075
aag aac gag ctg aag aac ggc agg ccc cgg tcc ggc gag acg cgg gcg 1123
gag ttc gag ggc cgc gtc gcg aag gag agc ttc gac gag gag aag ggc 1171
ttc cag cgg gcg cgt gag gtg gcg tcc gtc atg aac agg gcc ctg gag 1219
aac gcc cac gac gag agc gct tac ctc gac aac ctc aag aag gaa ctg 1267
gcg aac ggc aac gac gcc ctg cgc aac gag gac gcc cgt tcc ccg ttc 1315
tac tcg gcg ctg cgg aac acg ccg tcc ttc aag gag cgg aac gga ggc 1363
aat cac gac ccg tcc agg atg aag gcc gtc atc tac tcg aag cac ttc 1411
tgg agc ggc cag gac cgg tcg agt tcg gcc gac aag agg aag tac ggc 1459
gac ccg gac gcc ttc cgc ccc gcc ccg ggc acc ggc ctg gtc gac atg 1507
tcg agg gac agg aac att ccg cgc agc ccc acc agc ccc ggt gag gga 1555
ttc gtc aat ttc gac tac ggc tgg ttc ggc gcc cag acg gaa gcg gac 1603
gcc gac aag acc gtc tgg acc cac gga aat cac tat cac gcg ccc aat 1651
ggc agc ctg ggt gcc atg cat gtc tac gag agc aag ttc cgc aac tgg 1699
tcc gag ggt tac tcg gac ttc gac cgc gga gcc tat gtg atc acc ttc 1747
atc ccc aag agc tgg aac acc gcc ccc gac aag gta aag cag ggc tgg 1795
ccg tgatgtgagc g 1809

<210> 4
<211> 407
<212> PRT
<213> Streptoverticillium mobaraense

<400> 4

<210> 5
<211> 30
<212> DNA
<213> Artificial Sequence

<220>
<223> Description of Artificial Sequence:PCR primer for
amplification of transglutaminase gene from
S.cinnamoneum

<400> 5
tccgatgacc gggaaactcc tcccgccgag 30

<210> 6
<211> 30
<212> DNA
<213> Artificial Sequence

<220>
<223> Description of Artificial Sequence:PCR primer for
      amplification of transglutaminase gene from
      S.cinnamoneum

<400> 6
cggccagcct tgctccacct tggcgggggc                                        30

<210> 7
<211> 10
<212> PRT
<213> Streptoverticillium cinnamoneum

<400> 7
Gly Asp Gly Glu Glu Lys Gly Ser Tyr Ala
 1               5                  10